# EUROPEAN PATENT APPLICATION

(11) **EP 3 923 297 A1**
(43) Date of publication of application: **15.12.2021**
(21) Application number: 20179424.5
(22) Date of filing: 11.06.2020
(51) Int. Cl.: G16H 40/63, G16H 40/67

(54) **SIMULATION MODE FOR A MEDICAL DEVICE**

(71) Applicant: Koninklijke Philips N.V., 5656 AG Eindhoven (NL)
(72) Inventor: KNOTH, Thorsten, 5656 AE Eindhoven (NL); WOHLSCHLAGER, Markus Silvester, 5656 AE Eindhoven (NL)
(74) Representative: Philips Intellectual Property & Standards

(57) **Abstract**

A method and apparatus for implementing a simulation mode on a medical device, the medical device arranged in a normal operating mode to receive sensor input data, and to generate a sensory output based on the data. The method is based on taking control of the medical device to interrupt the flow of data between the sensor inputs and a monitoring processor of the medical device, and inputting to the processor, in place of the sensor input data, simulation signal data.

## Description

### FIELD OF THE INVENTION

This invention relates to a method and device for implementing a simulation or training mode on a medical device of a type which receives measurement sensor data and displays clinical parameter outputs.

### BACKGROUND OF THE INVENTION

The complexity of medical devices has increased significantly over the past decade due to general technical progress and also due to new diagnostic and therapeutic developments. In the face of such complexity, it is important that the operating personnel can operate the device reliably and interpret data outputs from the device correctly. To achieve this, training of the operator is essential.

In many countries, regulations require a certain minimum level of training. For example, in EU countries, the medical device regulation (MDR) makes it compulsory for each user of a medical device (greater than device class 1) to complete an introductory training session. For some devices, training must be repeated periodically. Furthermore, the management of a hospital must ensure that medical devices are operated only by trained personnel.

To optimize medical outcomes, it is also in the interest of a hospital to implement regular top-up and supplementary training. This can also have economic benefits in terms of improved medical performance statistics.

Training can include training in the operation and functions of the medical device, and also the interdisciplinary team workflow for performing a particular procedure. Several studies in obstetric care for example have found that the simulation of non-routine emergencies can improve the outcome for the patient.

For the most realistic training simulation, there is needed both a simulation patient, and simulated clinical parameters. A patient can be simulated by a mannequin, an actor or both. Currently, only three main ways are known to simulate clinical parameters, which all have disadvantages.

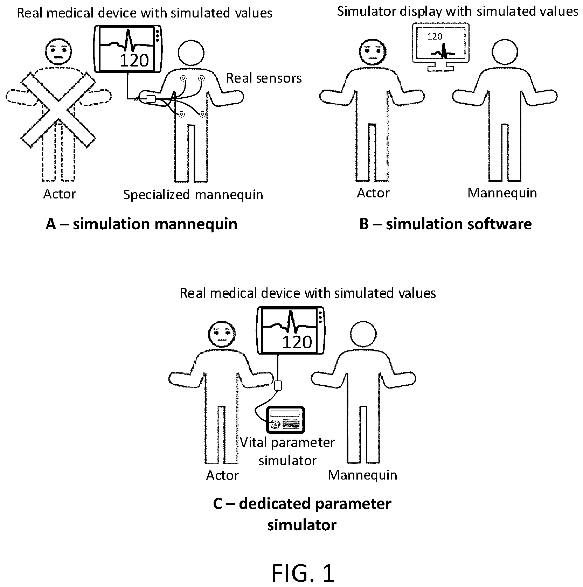

A first possibility is that a specialized mannequin can be used which is configured to deliver the clinical parameters itself. Some mannequins are able to simulate the mechanical action of physiological functions such as breathing, pulse rate and blood pressure, which can then be measured by normal medical sensors. This option is schematically depicted in Fig. 1A. Other mannequins simply comprise an integrated display that shows the physiological parameters. In either case, this option necessitates use of the specialized mannequin. The mannequin cannot for example be replaced with an actor.

The second possibility is that a dedicated simulator device is used to simulate the physiological parameters on a display, e.g. a display of a computer or a mobile device. This option is illustrated schematically in Fig. IB. This approach gives greater flexibility to switch between an actor and a mannequin.

The clinical parameters are simulated by software. For example, simulation software is known which operates using a pair of tablet computer displays. On one tablet, the simulated clinical parameters are displayed, and the other tablet is used as a control interface.

The third possibility is that one or more dedicated vital parameter simulators are used to generate simulated values for specific clinical parameters. These simulators can be plugged into a real medical device. This is illustrated schematically in Fig. 1C. Therefore, simulated measurement values are displayed on the real medical device. However, the simulators cannot be applied on the mannequin or actor.

A disadvantage of all known solutions is that the simulated data are generated artificially and the simulations are often not sufficiently realistic.

Furthermore, costs for known simulation solutions are high. Simulation software products must be provided with the associated hardware to run them which has a high combined cost. A specialized mannequin likewise can be very complex to manufacture and thus cost intensive. This high complexity and cost mean that simulation facilities often have to be hired, for example simulation training performed in an external simulation center. This is not ideal as the simulation environment is different from the actual clinical environment in which the equipment will be used.

In situ training is preferable since it provides a more realistic training experience.

Thus, it would be desirable to provide an improved medical device simulation, capable of offering a realistic, easy-to-handle, and inexpensive simulation.

### SUMMARY OF THE INVENTION

The invention is defined by the claims.

According to examples in accordance with an aspect of the invention, there is provided a method for implementing a simulation mode on a medical device,
the medical device being of a type configured in a standard mode to receive a set of one or more sensor inputs at a communication interface, each carrying a signal representative of a clinical parameter, to couple the sensor inputs to a processor, and the processor configured to generate a user output at a user interface, based on the one or more sensor inputs,
the method comprising:
detecting the set of sensor inputs connected at the communication interface;
interrupting the coupling of the sensor inputs to the processor, and
coupling to the processor in place of the sensor inputs a set of one or more simulation signals, each for simulating a sensor input carrying a particular clinical parameter, wherein the clinical parameters of the simulation signals is selected based at least in part on the detected sensor inputs connected at the communication interface.

The interruption decouples the physical sensor inputs from the device processor, and couples in their place a set of simulation signals which replicates clinical parameter signals. These may simulate exactly the set of clinical parameters carried by the connected sensor inputs, or may at least partly differ.

Thus, embodiments provide a method for transforming any medical device, e.g. a device having patient monitoring functionality, into a simulator device based on taking control of the data connection between the measurement input ports of the device and the normal processing operations of the device. The method effectively overwrites the real measured data with simulated data. As a result, the medical device (e.g. patient monitoring device) behaves in the same way as it does when treating real patient data, with full normal functionality, but displays simulated physiological parameters instead of real ones.

The resulting simulation is thus highly realistic since the simulation is run on the actual medical device that the trainee will be using in their clinical practice. It can be also be run in the same clinical environment in which the equipment will be used, further adding to the realism of the simulation. Furthermore, this approach substantially avoids the need to buy expensive dedicated simulation hardware since all of the simulation outputs can be provided using the user output means of the medical device itself. This saves cost and also space in the hospital facility.

This offers flexibility in transforming any available medical device (receiving measurement data inputs and generating clinical data outputs) used for real medical practice temporarily in to a simulator.

In certain embodiments, in the simulation mode, the device is also decoupled from any monitoring or alarm network to which it is connected so that it is isolated and all functionality of the device can be explored without risk of triggering alarms. In some cases, a connected monitoring network may be operable to distinguish between real measurement data and simulated data. In these cases, the device may be kept coupled to the monitoring network even in the simulation mode.

The simulation signals may be recorded sets of sensor signals captured from real historical patients.

There may be stored a plurality of simulation datasets, each corresponding to a particular clinical scenario. Thus, a user can be trained using historical data from different clinical cases, these being run on the same monitoring device that the user will be operating in actual practice.

The simulation signals can be supplied in real (run) time, so that they effectively replicate receipt at the device of actual input sensor signal data.

The execution of the simulation method may be triggered by a control input, for example by a user input command, responsive to which the medical device is switched from the standard operating mode to the simulation mode. The control input may be received for example from a user via a user interface of the medical device, and/or from an external device such as mobile communication device to which the medical device is operatively coupled, or from a user input means comprised by an external hardware module which is configured to execute the method.

The simulation method may be implemented by a dedicated hardware module, such as a peripheral unit which operatively couples with the medical device in use, or may be implemented by a controller integrated in the medical device itself in some examples.

The processor of the medical device mentioned above is for example a monitoring processor. It may be the normal processor of the medical device used to configure the receipt of sensor input data and output of the clinical parameter information based on the sensor inputs.

The medical device may be configured to receive the sensor inputs via connection of corresponding measurement devices to the communication interface. The measurement devices are for example peripheral measurement devices such as sensors, transducer units, and/or imaging devices. These can be the real measurement devices that are used during actual operation, adding to realism of the simulation. Detecting the sensor inputs connected at the interface may comprise detecting the clinical parameters carried by the sensor inputs and/or detecting which measurement devices are connected to the connection interface.

The standard or normal operating mode is for example a patient monitoring mode.

In some embodiments, the clinical parameters simulated by the simulation signals may include at least each of the clinical parameters of the detected sensor inputs connected at the connection interface. In this case, the clinical parameters of the simulation signals match those of the sensor inputs connected at the communication interface. The simulation signals may include further simulated clinical parameters in addition to these in some examples.

Hence, in this case, the signals which are simulated are configured to mirror the sensor inputs connected by the user to the communication interface. This hence provides a highly realistic simulation, since the simulated signals which are output via the user interface change in dependence upon which measurement devices the user connects to the medical device.

The method may further comprise interrupting a communication channel between the monitoring device and at least one or more components of a medical facility monitoring network. The monitoring network may for example be a hospital facility network. This may for example include an alarm network. This way, the simulation can run in isolation from any surrounding monitoring ecosystem, so that all functionality of the medical device can be safely utilized as part of the simulation without risk of triggering alarms or clinical actions on the monitoring network.

In some cases, at least a subset of components of the monitoring network may be operable to distinguish between real measurement data and simulated data, and thus are able to handle receipt of simulated data, and perform a monitoring network simulation function using the simulated data. Thus according to some embodiments, the method may comprise interrupting a communication channel with only monitoring network components which do not include a simulation function for receiving simulation data.

According to one or more embodiments, supplying the simulation signals may comprise communicating with a datastore, the datastore storing reference simulation data for a plurality of different simulation signals. Supplying the simulation signals may comprise generating the simulation signals based on simulation data in the datastore. Alternatively, supplying the simulation signals may simply comprise outputting to the processor the simulation measurement values stored in the datastore for the respective simulation signal.

The datastore may be stored on a memory of the medical device or on a separate external memory or datastore. It may be a remote datastore accessed via a network or Internet connection in some examples.

The simulation data used as the basis for the simulation signals may include historical sensor signal data recorded for one or more prior patients over a time window. Thus, the simulations can be based in this case on real historical patient cases, therefore maximizing the realism of the simulation.

The simulation data may include a plurality of simulation data subsets, each data subset comprising simulation signal data for simulating a set of clinical parameters for a particular clinical scenario, over a time window for that clinical scenario.

In some examples, a start time of the simulation within the time window of each simulation dataset can be selected as desired. For example, the control input may include an indication of a start point within the time window of the stored simulation data. The method may comprise supplying simulation data to the processor corresponding to the portion of the simulation data falling after this time point in the time window. This may be supplied in real time as real-time simulated measurement data.

The data of the dataset before this start point may in some examples be further supplied to the processor as historical trend data, for output at the user interface (e.g. display unit) as previous measurement data.

In some examples, the method may further include functionality to selectively implement a further (demonstration) mode on the device for demonstrating medical device functionality. The demonstration mode also comprises interrupting the coupling of the sensor inputs to the processor. The demonstration mode may further comprise communicating with the processor to cause the processor to execute one or more pre-stored demonstration control routines. The method may comprise selectively switching between any of: the simulation mode, the demonstration mode, the standard operating mode of the device.

According to one set of embodiments, the medical device may be a patient monitor device, for example a fetal monitor device. It may be adapted to monitor at least a fetal and/or maternal heart rate for example.

The sensor inputs may be received for example from any clinical parameter measurement device, sensor, or detector. The sensor inputs may include ultrasound data inputs in some examples, for example from one or more ultrasound transducer units. The ultrasound transducer unit may be an ultrasound-based clinical parameter monitor in some examples, e.g. a tocodynamometer, fetal heart rate detector, and/or a hemodynamic parameter monitor. The ultrasound transducer unit may be an ultrasound imaging probe in other examples.

The clinical parameters may be for example physiological parameters such as vital signs (e.g. pulse rate, blood oxygen saturation, breathing rate, blood pressure, cardiac output), and/or anatomical parameters such as dimensions or volumetric capacities of bodily organs such as the lungs or heart chambers.

The user interface may comprise a display. The user output may be a visual or display output. The user interface may be configured to provide other sensory outputs such as acoustic output.

According to one or more embodiments, the communication interface may comprise a set of physical sensor input ports accessible at an exterior face of the medical device.

Detecting the set of connected sensor inputs may comprise detecting which sensor inputs are physically connected to a sensor input port, e.g. detecting what sensor input connectors are plugged in to the communication ports. However, wireless connection ports are also a possibility in other examples.

De-coupling the sensor inputs from the processor may comprise decoupling a data connection between at least a subset of the sensor input ports and the processor. This may be done digitally in some examples.

According to one advantageous set of embodiments, the method may comprise receiving a control input from an external device and configuring the signal interruption and simulation signal supply based on the control commands. The external device may be a portable handheld device such as a mobile communication device, e.g. a mobile phone (smartphone) or tablet computer.

The set of simulation signals provided to the processor may be configured based on the control input.

According to this set of embodiments, parameters and operating settings of the simulation mode can be controlled by an external device such as a mobile communication device. The external device is external to the medical device. The external device may be operated by a trainer or supervisor who can use the external device to control parameters of the simulation for the trainee or user.

The selection of clinical parameters provided to the processor may be controllable with the external device, and/or the particular clinical scenario which is being simulated may be controlled, the clinical scenario having a certain set of stored simulation signals associated with it. Selective activation and deactivation of the simulation mode may be controllable by the external device, for example toggling between a standard operating mode and a simulation mode.

There may be multiple different simulation modes which can be switched between.

For example, according to one or more embodiments, the method may include selectively implementing a further simulation mode, for example responsive to detection of a pre-determined control signal from the external device, and wherein, in the further simulation mode, the set of clinical parameters of the simulation signals is selected based on one or more control inputs received from the external device. The set of clinical parameters may be selected based partly on the one or more control inputs, or based fully on the one or more control inputs.

The method may comprise a step of establishing a communication channel with the external device via a further communication interface, and preferably wherein the establishing communication comprises exchanging one or more handshake messages. This step may be an initial step, for example performed in advance of detecting the connected sensor inputs and/or in advance of supplying the simulation signals.

The initial step may comprise sending a handshake message to the medical device to open a communication channel between the external device and the medical device. The one or more handshake messages include authentication information for establishing a secure communication channel. For example, the one or more handshake messages may include a secure authentication message to authenticate the external device. This may comprise a step of obtaining user credentials by means of a user input means.

The further communication interface may include a local wireless network interface. The communication channel may be via a local wireless network server, for example a Wi-Fi server. However, other wireless communication protocols may alternatively be used, such as Bluetooth, radio frequency (RF) communication, Near Field Communication (NFC) or any other wireless communication means.

In some examples, the establishing communication may comprise communicating with a web server via a wireless LAN server. For example, a hardware unit may be provided for performing the method, for connecting to the medical device, and comprising a Wi-Fi connection interface. The hardware unit may be configured to perform a Wi-Fi server function.

The network server may facilitate secure connection via an authentication network-page, e.g. a log-in intranet page via which authentication credentials can be entered and the secure connection established. This provides a secure connection interface, since connection with the medical device can only be done locally, in wireless connection range of the medical device.

However, this is not essential and in other examples connection could be established via an internet connection.

Examples in accordance with a further aspect of the invention provide a computer program product comprising code means, configured when run on a processor, to cause the processor to perform the method in accordance with any example or embodiment outlined above or described below, or in accordance with any claim of this application.

Examples in accordance with a further aspect of the invention provide a controller, arranged for communicating with a medical device, for implementing a simulation mode on the medical device, the medical device configured in a normal operating mode to receive one or more sensor inputs at a communication interface, each carrying a signal representative of a clinical parameter, supply the sensor inputs to a processor, and to generate a user output using the processor,
the controller configured to:
communicate with the medical device to cause the medical device to interrupt the coupling of the sensor inputs to the processor of the medical device;
supply to the processor, in place of the set of sensor input signals, a set of one or more simulation signals, each for simulating a sensor input carrying a particular clinical parameter, wherein the clinical parameters of the simulation signals is selected based at least in part on the sensor inputs connected at the communication interface.

The controller may comprise an input/output for receiving and transmitting data when the controller is communicatively coupled with the medical device for example.

In accordance with one or more embodiments, the controller may be integrated in a peripheral hardware module, the hardware module comprising a connection interface, and adapted to operatively couple to the medical device via the connection interface. Thus a further aspect may provide a hardware module comprising a controller in accordance with any example or embodiment outlined above or described below, or in accordance with any claim of this application, the hardware module comprising a connection interface, and adapted to operatively couple to the medical device via the connection interface.

However, this arrangement is not essential. In alternative examples, the control unit may be integrated in the medical device for example.

Examples in accordance with a further aspect of the invention provide a system, comprising:
a medical device configured in a standard mode to receive a set of one or more sensor inputs at a communication interface, each carrying a signal representative of a clinical parameter, couple the sensor inputs to a processor, and the processor to generate a user output at a user interface, based on the one or more sensor inputs; and
a controller in accordance with any example or embodiment outlined above or described below, or in accordance with any claim of this application.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 schematically illustrates example simulation arrangements not in accordance with embodiments of the invention;
Fig. 2 schematically depicts implementation of an example simulation mode on an example medical device;
Fig. 3 schematically depicts implementation of a further example simulation mode on an example medical device;
Fig. 4 schematically depicts components of an example apparatus for implementing a simulation mode on a medical device; and
Fig. 5 schematically depicts an example process for establishing a secure communication channel between an external device and a hardware unit configured to implement a simulation mode on a medical device.

### DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

The invention provides a method and apparatus for implementing a simulation mode on a medical device, the medical device arranged in a normal operating mode to receive sensor input data, and to generate a sensory output based on the data. The method is based on taking control of the medical device to interrupt the flow of data between the sensor inputs and a monitoring processor of the medical device, and inputting to the processor, in place of the sensor input data, simulation signal data which simulates sensor inputs. The processor treats the simulation signal data in the same way it would treat true measured data, and thus the processing and user output functions of the medical device operate exactly as they would in real practice, were the simulated data is true data. This way, a user or operator can simulate different clinical scenarios with the medical device as a form of training.

In some embodiments, the configuration of the simulation data (e.g. which clinical parameters are simulated, and which clinical scenario to simulate) can be configured by a further operator, for example by means of a portable unit which connects to the medical device or connects to external hardware which implements the method.

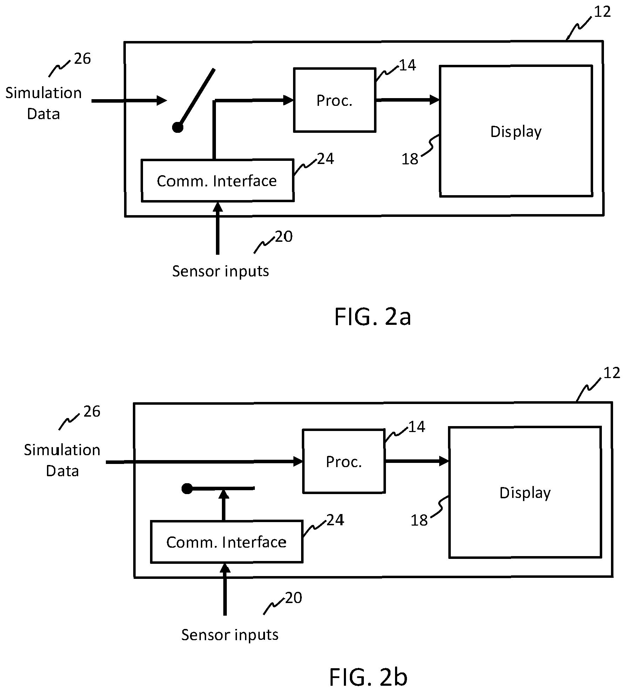

An example method in accordance with one or more embodiments will now be described with reference to Fig. 2a and Fig. 2b. The method is a computer-implemented method. It may be implemented by a controller or control unit for example.

The method is for implementing a simulation mode on a medical device. To illustrate the concept of the method, Figs. 2a and 2b schematically depict an example medical device 12 to which the method may be applied.

The medical device 12 is configured with a standard operating mode in which it is configured to receive a set of one or more sensor inputs 20 at a communication interface 24, each carrying a signal representative of a clinical parameter, and to couple the sensor inputs to a processor 14. The processor is configured to generate a user output at a user interface, based on the one or more sensor inputs. The user interface in this example comprises a display 18, and the processor is adapted to provide a visual output representative of the one or more clinical parameters at the display output.

The method comprises:
detecting the set of sensor inputs 20 connected at the communication interface 24
interrupting the coupling of the sensor inputs 20 to the processor 14, and
coupling to the processor 14 in place of the sensor inputs 20 a set of one or more simulation signals 26, each for simulating a sensor input carrying a particular clinical parameter, wherein the clinical parameters of the simulation signals is selected based at least in part on the detected sensor inputs 20 connected to the communication interface 24.

Fig. 2a shows the medical device 12 in the standard operating mode, in which the sensor inputs 20 at the communication interface 24 are communicatively coupled to a data input of the processor. Fig. 2b shows the medical device in the simulation mode, with the communication interface decoupled from the processor and the simulation signals 26 coupled to the processor data input in place of the sensor inputs 20.

Thus, embodiments provide a method for transforming any medical device having patient monitoring functionality into a simulator device based on taking control of the data connection between the measurement input ports of the device and the normal processing operations of the medical device. The method effectively overwrites the real measured data with simulated data. As a result, the medical device (e.g. patient monitoring device) behaves in the same way as it does when treating real patient data, with full standard functionality, but displays simulated physiological parameters instead of real ones.

The resulting simulation is thus highly realistic since the simulation is run on the actual medical device that the trainee will be using in their clinical practice. It can be also be run in the same clinical environment in which the equipment will be used, further adding to the realism of the simulation. Furthermore, this approach substantially avoids the need to buy expensive dedicated simulation hardware since all of the simulation outputs can be provided using the user output means of the medical device itself. This saves cost and also space in the hospital facility.

Thus, in effect, any suitable monitoring system can be converted into a simulation system. This permits a physician or other healthcare professional to simply walk into a patient room which is currently not occupied, switch the medical device to the simulation mode, and begin a simulation directly.

The execution of the simulation method may be triggered by a control input, for example by a user input command, responsive to which the medical device is switched from the standard operating mode to the simulation mode. The control input may be received for example from a user via a user interface of the medical device, and/or from an external device such as mobile communication device to which the medical device is operatively coupled, or from a user input means comprised by an external hardware module which is configured to execute the method.

The simulation method may be implemented by a dedicated hardware module, such as a peripheral unit which operatively couples with the medical device in use, or may be implemented by a controller integrated in the medical device itself in some examples.

In an advantageous set of embodiments, the method may comprise receiving a control input from an external device and configuring the signal interruption and simulation signal supply based on the control commands. The external device may be a portable handheld device such as a mobile phone (smartphone) or tablet computer for example.

The set of simulation signals provided to the processor may be configured based on the control input.

According to this set of embodiments, parameters and operating settings of the simulation mode can be controlled by an external device such as a mobile communication device. The external device is external to the medical device. The external device may be operated by a trainer or supervisor who can use the external device to control parameters of the simulation for the trainee or user.

The selection of clinical parameters provided to the processor may be controllable with the external device, and/or the particular clinical scenario which is being simulated may be controlled, the clinical scenario having a certain set of stored simulation signals associated with it. Selective activation and deactivation of the simulation mode may be controllable by the external device, for example toggling between a standard operating mode and a simulation mode.

Furthermore, according to one or more embodiments, the particular data points, and or pattern of data points in the simulation signals may be controllable with the external device.

The method may comprise a step of establishing a communication channel with the external device via a further communication interface, and preferably wherein the establishing communication comprises exchanging one or more handshake messages. This step may be an initial step, for example performed in advance of detecting the connected sensor inputs and/or in advance of supplying the simulation signals.

In this way, activation and configuration of a simulation can be controlled by an operator with a standard mobile phone or other mobile communication device. Details of this feature will be discussed further later in this disclosure.

The communication channel may be a wireless communication channel in advantageous examples, although a wired communication channel is also possible. The further communication interface may comprise for example a local wireless network interface. Other example wireless communication interfaces may include Bluetooth, radio frequency (RF), or near field communication (NFC) interfaces for example.

In some embodiments, the clinical parameters of the simulation signals may include at least each of the clinical parameters of the detected sensor inputs connected at the connection interface. In this case, the clinical parameters of the simulation signals match those of the sensor inputs connected at the communication interface. The simulation signals may include further simulated clinical parameters in addition to these in some examples.

Hence, in this case, the signals which are simulated are configured to follow the sensor input connected by the user to the communication interface. This hence provides a highly realistic simulation, since the simulated signals which are output via the user interface change in dependence upon which measurement devices the user connects to the medical device.

In some embodiments, the method may comprise receiving a user input (e.g. from the external device) for guiding adjustment or configuration of the simulation signals. For example, the selection of simulated clinical parameters which are provided to the medical device processor may be determined by default based on which sensor inputs are plugged into the communication interface by the user (for example exactly mirroring the clinical parameters of the connected sensor inputs). However, an operator may have the ability to change or adjust the particular values or signal patterns of those simulation signals which are provided.

This approach is the most realistic simulation mode, since the signals input to the medical device follow the actions of the user (trainee) in connecting different sensor hardware to the medical device.

However, an alternative simulation mode is possible (referred to herein as an open simulation mode), wherein the operator has the capability also of selecting which simulation signals are provided to the medical device processor (i.e. which simulated clinical parameters are provided). Thus, in this mode, an operator can choose to supply a particular simulated clinical parameter without the user (trainee) having connected to the communication interface the corresponding clinical parameter sensing hardware. This mode can be helpful for example for introductory training or demonstrations to a third party.

The selection of simulation signals provided to the processor may be based in part on the set of sensor inputs connected to the user interface and in part on a control input from the operator, e.g. via the external device. For example, the operator may choose to add additional simulated clinical parameters, to augment the clinical parameters of the set of sensor inputs.

In accordance with one or more embodiments, the method may further comprise selectively implementing a further simulation mode, wherein, in the further simulation mode, the set of clinical parameters of the simulation signals is selected based on one or more control inputs received from the external device. The selection of simulation signals may be based in part on the set of sensor inputs connected to the user interface and in part on the control input from the operator. Alternatively, it may be based fully on the control inputs. The switching to the further simulation mode (and back to the first simulation mode) may be performed responsive to detection of a pre-determined control signal from the external device.

According to any of the above examples, where the operator does adjust or configure the simulated clinical parameters, this can be done in different ways.

In some examples, the operator may have the capability to control the value of each clinical parameter manually.

In some examples, the operator may have the capability to select a particular pre-stored evolution pattern for a clinical parameter, upon selection of which the particular parameter values as a function of time are automatically generated and output to the processor.

In some examples, a combination of both capabilities may be provided, wherein a particular parameter time-evolution pattern is selectable, but wherein individual values of the same clinical parameter can be adjusted or changed at any given time, in real time.

The simulation signals provided to the processor may be recorded sets of sensor signals captured from real historical patients. There may be stored a plurality of simulation datasets, each corresponding to a particular clinical scenario. Thus, a user can be trained using historical data from different clinical cases, these being run on the same monitoring device that the user will be operating in actual practice.

In particular, according to one or more embodiments, supplying the simulation signals may comprise communicating with a datastore, the datastore storing reference simulation data for a plurality of different simulation signals. Supplying the simulation signals may comprise generating the simulation signals based on simulation data in the datastore. Alternatively, supplying the simulation signals may simply comprise outputting to the processor the simulation measurement values stored in the datastore for the respective simulation signal.

The simulation data used as the basis for the simulation signals may include historical sensor signal data recorded for one or more prior patients over a time window. Thus, the simulations can be based in this case on real historical patient cases, therefore maximizing the realism of the simulation.

The simulation data may include a plurality of simulation data subsets, each data subset comprising simulation signal data for simulating a set of clinical parameters for a particular clinical scenario, over a time window for that clinical scenario.

In some examples, a start time of the simulation within the time window of each simulation dataset can be selected as desired. For example, the control input may include an indication of a start point within the time window of the stored simulation data. The method may comprise supplying simulation data to the processor corresponding to the portion of the simulation data falling after this time point in the time window. This may be supplied in real time as real-time simulated measurement data. The data of the dataset before this start point may in some examples be further supplied to the processor as historical trend data, for output at the user interface (e.g. display unit) as previous measurement data.

For example, a typical simulation may last 10 to 15 minutes. However, a clinical scenario, e.g. a birth, may last several hours or even days. It may be desirable to simulate only a portion of the full clinical scenario. By enabling selection of a defined start point within the scenario, and outputting previous simulation data as trend data, this simulates a particular portion of the clinical scenario, starting after the defined start point.

The datastore storing the historical data may be facilitated by a local memory of the medical device or by a separate external memory or datastore. It may be a remote datastore accessed via a network or Internet connection in some examples.

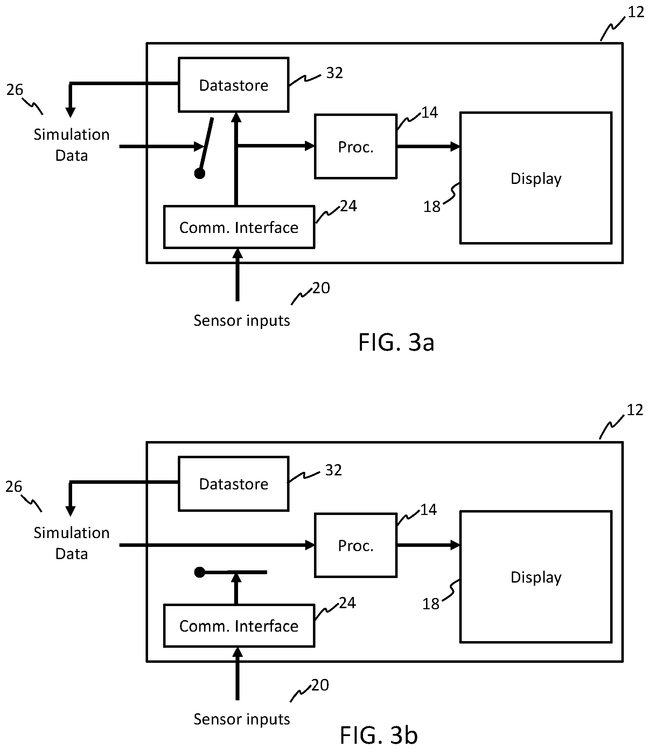

By way of example, Fig. 3 schematically depicts one example in which the datastore is comprised locally by the medical device.

Fig. 3a shows the example medical device 12 in the standard operating mode (patient monitoring mode). Fig. 3b shows the example medical device in the simulation mode.

The example illustrated in Fig. 3 is the same in all respects as that of Fig. 2, except for the inclusion within the medical device 12 of a datastore 32 which stores reference simulation data for a plurality of different simulation signals as discussed above. In this example, the reference simulation data is historical sensor signal data recorded for one or more prior patients over a time window.

In the standard monitoring mode (Fig. 3a), the sensor input 20 data is routed both to the monitoring processor 14 and to the datastore 32, and the datastore records a copy of the sensor signal 20 data for the patient in question over time. This may build up a set of sensor signal waveforms for the patient over the measurement period. An operator of the medical device may label the set of reference sensor signals in accordance with the particular clinical scenario to which they correspond (e.g. the particular clinical status of the patient at the time they were recorded including the medical condition the patient was suffering from, and their level of acuity for instance). This recording of data can be done for each patient connected to the medical device, so that a collection of reference data subsets are accumulated, each comprising a set of sensor signal data for a particular real patient in a particular clinical condition.

In the simulation mode (Fig. 3b), this stored reference data is retrieved and used in the provision of the simulation data. The recorded patient sensor signals may either be used directly as the provided simulation data 26 or these recorded signals may be adjusted or modified at least partially to provide the simulation signals.

The simulation data from the datastore 32 may be supplemented with additional simulation data from one or more further sources in some examples. For example, additional simulation data may be retrieved from a remote datastore, such as a remote (e.g. central) server. The remote central server might store historical sensor signal data captured from multiple different medical devices for example.

Although in the example of Fig. 3, the simulation signals are compiled using historical sensor signal data from previous patients, this is not essential. In other examples, simulation data may be artificial, for instance generated manually in advance so as to simulate one or more different clinical scenarios. Also, although the simulation data is stored in, and retrieved from, a datastore 32 local to the medical device, this is also not essential. The datastore may be located outside of the medical device. For example, a datastore may be integrated in a peripheral hardware unit configured to implement the simulation mode on the medical device. A datastore may be comprised by a remote computer or server in some examples.

In accordance with one or more embodiments, the method may comprise executing one or more pre-stored training routines or exercises. A training routine or exercise may have one or more associated simulation signals stored in a dataset, and wherein execution of the training routine or exercise comprises supplying the associated simulation signals in a predefined pattern or order to the processor of the medical device. Each training routine or exercise may be associated with a particular clinical scenario or pathology for example. The training routine or exercise which is executed may be controllable, for example by an external device from which a control input is received as part of the method. Alternatively, it may be selected by a user via a user interface coupled with the medical device or with a hardware unit which implements the simulation method.

The training routines or exercises may additionally or alternatively be for training a user in use of one or more functions or features of the medical device. The medical device is decoupled from the sensor inputs and preferably further decoupled from any monitoring or alarm network to which it is connected (e.g. if that monitoring alarm or network is not configured to handle simulation data). This way, the user can practice all functions and features of the medical device without risk of triggering any inadvertent alarms or actions within the medical institution.

In some examples, the method may further include functionality to selectively implement a further (demonstration) mode on the device for demonstrating medical device functionality. The demonstration mode also comprises interrupting the coupling of the sensor inputs to the processor. The demonstration mode may further comprise communicating with the processor to cause the processor to execute one or more pre-stored demonstration control routines. The method may comprise selectively switching between any of: the simulation mode, the demonstration mode, the standard operating mode of the device.

The medical device may be configured to receive the sensor inputs via connection of corresponding measurement devices to the communication interface. The measurement devices are for example peripheral measurement devices such as sensors or ultrasound transducers. Detecting the sensor inputs connected at the interface may comprise detecting the clinical parameters carried by the sensor inputs and/or detecting what measurement devices are connected to the connection interface.

This offers a significant advantage over existing simulation devices, since it permits the simulation to be integrated with the natural interaction of the user with the medical device. For example, with known simulation systems, user interaction with a user interface of the medical device is not possible, nor the interaction with the measurement hardware itself, e.g. plugging or unplugging of peripherals and accessories.

The solution proposed according to embodiments of the present invention allows any patient monitoring system, in any environment, to be used as a simulator. This offers the advantage that the trainee user can train using the same hardware that they will be using in practice.

The simulation approach provided according to embodiments of the present invention also offers advantages over mannequin based simulation methods. As discussed above, even the most sophisticated simulation mannequins are not able to simulate all clinical parameters. By instead using pre-stored simulation data to supply the simulation signals to the medical device processor, it is possible to simulate any desired clinical parameter measurement or signal.

According to embodiments of the present invention, greater flexibility is provided. The separation method can be performed with use of an actor playing the role of a patient being examined or treated with the medical device, or alternatively a simulation mannequin may be used to supplement the simulation signals provided by the simulation method of the present invention. An actor may make the situation scenario more realistic, since a certain amount of interaction with the trainee is possible. A hybrid simulation approach is also possible in which a combination of an actor and a mannequin is used.

As discussed above, in accordance with one or more embodiments of the invention the simulation method may be implemented by a peripheral hardware unit which is arranged to operatively connect with the medical device. For example, the peripheral hardware unit may comprise a controller which is configured to implement the simulation method on the medical device when connected with the medical device.

Thus, according to a further aspect of the invention, there is provided a controller, arranged for communicating with a medical device for implementing a simulation mode on the medical device, the medical device configured in a normal operating mode to receive one or more sensor inputs, each carrying a signal representative of a clinical parameter, supply the sensor inputs to a processor, and to generate a user output using the processor.

The controller is configured to:
communicate with the medical device to cause the medical device to interrupt the coupling of the sensor inputs to the processor of the medical device; and
supply to the processor, in place of the set of sensor input signals, a set of one or more simulation signals, each for simulating a sensor input carrying a particular clinical parameter, wherein the clinical parameters of the simulation signals is selected at least in part on the sensor inputs connected at the communication interface.

In accordance with one or more embodiments, the controller may be integrated in a peripheral hardware module, the hardware module comprising a connection interface, and adapted to operatively couple to the medical device via the connection interface. Thus, a further aspect may provide a hardware module comprising a control unit in accordance with any example or embodiment outlined above or described below, or in accordance with any claim of this application, the hardware module comprising a connection interface, and adapted to operatively couple to the medical device via the connection interface.

However, this arrangement is not essential. In alternative examples, the control unit may be integrated in the medical device for example.

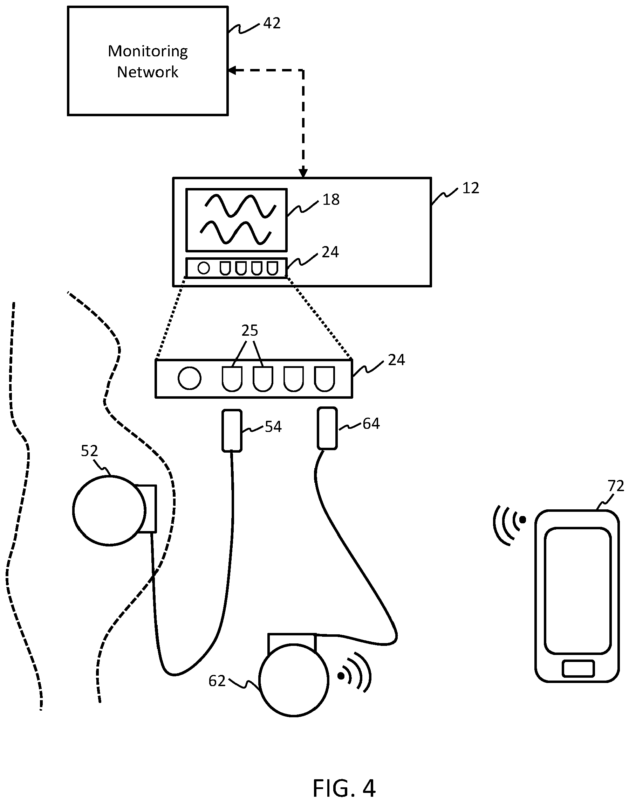

An example arrangement in which the simulation mode is implemented by a peripheral hardware module will now be discussed with reference to Fig. 4.

A medical device 12 is shown, comprising a display 18 and a communication interface 24. The communication interface 24 comprises a set of physical sensor input ports 25 accessible at an exterior face of the medical device. For example, the communication interface may comprise a bus system.

The sensor input ports 25 are for receiving physical connectors 54 of peripheral medical sensor units 52, for receiving sensor input data from the medical sensor units 52. Detecting which sensor inputs are connected at the communication interface 24 may comprise detecting what medical sensors are physically connected at the connection ports 25.

In some examples, the medical sensor units 52 may include one or more ultrasound transducer units. This can be useful for example in obstetrics for monitoring a heart rate of the fetus and/or of the mother. This will be explained in further detail to follow. In accordance with the example of Fig. 4, the medical device is also communicatively linked with a medical institution monitoring network 42, the monitoring network incorporating an alarm function for triggering an alarm in the event that one or more clinical parameters being monitored by the medical device 12 exceed one or more predefined thresholds. The monitoring network may also incorporate a patient management and data archiving function in some examples.

In the example of Fig. 4, the simulation method is implemented by a peripheral hardware unit 62 (referred to herein as a peripheral simulation unit) which is adapted to operatively couple with the medical device 12 by means of one of the connection ports 25 comprised by the communication interface 24. The connection port 25 may provide both a power supply to the simulation unit 62 and provide a data connection with the simulation unit 62.

When connected to the medical device 12, the peripheral simulation unit 62 is operable to implement the simulation method in accordance with any of the examples or embodiments discussed above or below. Activation of the simulation method may be controllable by a user interface built into the peripheral unit 62, or by means of an external device 72 arranged to communicatively couple with the peripheral unit 62, for example via a wireless network connection.

In accordance with one or more examples, activation of the simulation mode may be possible only when the medical device 12 is switched locally into a particular operation mode, or otherwise unlocked in some way. For example, the medical device may need to be unlocked by entry of a password directly into the user interface of the medical device before the simulation mode can be executed. Thus, switching the system to the simulation mode is protected and cannot be performed by remote actions alone, but involves a step which must be performed directly on the device itself. This provides for patient security, since the medical device cannot be deactivated from a standard monitoring mode remotely without a local operator deliberately unlocking it.

Upon activation of the simulation mode, the medical device 12 may be further disconnected from the monitoring network 42. Thus, all functionality of the medical device can be used and explored without triggering any alarms on the monitoring network.

In some cases, at least a portion of the monitoring network may be operable to distinguish between real measurement data and simulated data, and thus is able to handle receipt of simulated data, and perform a monitoring network simulation function using the simulated data. Thus, according to some embodiments, the method may comprise interrupting a communication channel with only monitoring network components which are not operable to handle simulation data.

According to one or more embodiments, a system may be provided which comprises the medical device 12, in combination with a second display (not shown in Fig. 4) operatively coupled to the medical device, and arranged to mirror the display output 18 of the medical device. This second display can be used by an instructor to monitor the output of the medical device which the trainee user is seeing.

In accordance with some examples, the simulation data may be communicated both to the processor of the medical device 12 and also to a remote server, connected to the medical device for example by a local area network connection, the remote server arranged to record the simulated signal data for the particular simulation session. This therefore replicates the function of recording an actual patient's clinical parameter signals as part of a medical history recording system. This means that after the simulation, the trainee user can review the simulated signals on the remote server in the way that they would in a realistic scenario for reviewing the medical history of an actual patient. This therefore further enhances the simulation realism. The remote server may incorporate a patient management function in some examples. The remote server may incorporate a data archiving function in some examples.

By way of example, one particularly advantageous application for embodiments of the present invention is the field of fetal monitoring.

In obstetric care, there are irregular occurrences of serious and unpredictable emergency situations. Some clinical scenarios for example are very rare, but have a high morbidity. Due to their rarity, it is difficult for medical staff to gain practical experience in handling them. Thus, a simulation method which permits simulating such rare scenarios can significantly improve patient outcome, by ensuring that all clinical staff are trained to handle these scenarios.

Within the field of fetal monitoring, an important medical sensor unit is the cardiotocograph (CTG). A CTGunit performs a dual role of monitoring fetal and maternal heart rate and also monitoring uterine contractions. Typically, a CTG unit may incorporate one or more ultrasound transducers for monitoring the heart rate, and, in some cases, additionally another sensing modality for monitoring uterine contractions, for example a strain gauge or an optical sensor.

Thus, in accordance with an advantageous set of embodiments, the simulation mode may be for simulating a CTG sensor input, including a simulated fetal and/or maternal heart rate signal, and a simulated tocodynamometer signal (uterine contraction signal). The simulated fetal and/or maternal heart rate signal may be in the form of one or more simulated ultrasound signals, from which the processor of the medical device derives a fetal heart rate and/or maternal heart rate.

Thus, the simulated data may include simulated ultrasound transducer data. As discussed above, this may comprise historical ultrasound transducer data recorded for previous obstetric patients, for example for previous patients in one or more of the rare high morbidity clinical scenarios.

According to one advantageous set of embodiments, the method of implementing the simulation mode on the medical device may comprise receiving a control input from an external device and configuring the signal interruption and simulation signal supply based on the control commands. The external device may be a portable handheld device such as a mobile communication device, e.g. a mobile phone (smartphone) or tablet computer.

The method may comprise a step of establishing a communication channel with the external device via a further communication interface, and preferably wherein the establishing communication comprises exchanging one or more handshake messages. This step may be an initial step, for example performed in advance of detecting the connected sensor inputs and/or in advance of supplying the simulation signals.

The initial step may comprise sending a handshake message to the medical device to open a communication channel between the external device and the medical device. The one or more handshake messages include authentication information for establishing a secure communication channel. For example, the one or more handshake messages may include a secure authentication message to authenticate the external device. This may comprise a step of obtaining user credentials by means of a user input means.

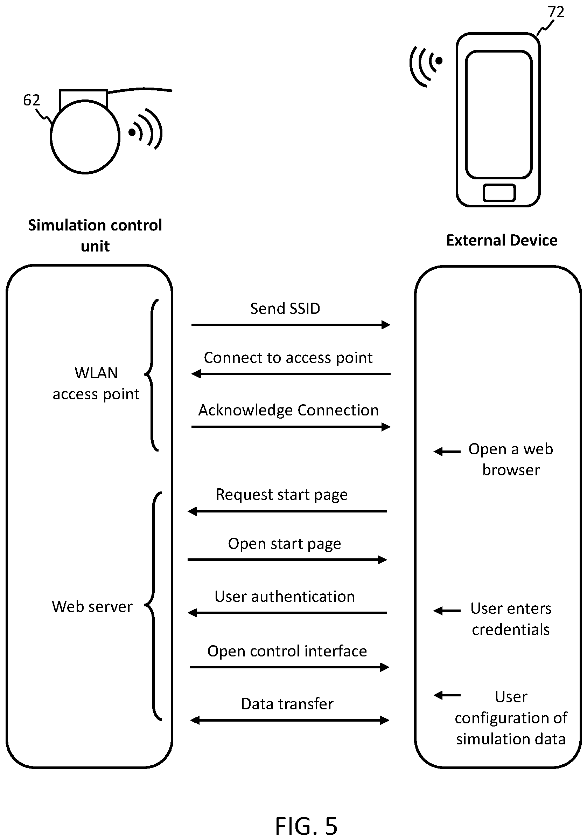

Fig. 5 schematically outlines a series of steps for establishing a secure communication channel with an external device such as a mobile phone, for receiving a control input from the external device. This is illustrated in Fig. 5 with reference to a peripheral hardware module ("simulation control unit") configured to implement the simulation method, as discussed above with reference to Fig. 4. However, it is noted that the same procedure can be applied regardless of the particular means by which the method is implemented. For example, the same procedure may be followed where the simulation mode is implemented by software installed on the medical device.

In this example, the further communication interface comprises a local wireless network interface. In particular, the communication channel is facilitated by a wireless local area network (WLAN) server, for example a Wi-Fi server. Wireless communication may be preferable to wired communication for reasons of electrical safety.

In the example of Fig. 5, the peripheral simulation unit 62 comprises a wireless communication module, which allows the simulation unit 62 to act as a wireless LAN access point to which an external device 72 can connect as a client. This offers the most secure approach, since all communication is confined to a direct communicative coupling between the peripheral simulation unit 62 and the external device; a wider network environment (which could be more vulnerable to hacking or unauthorized access) is not needed. A further advantage is that any external device having a wireless network communication function, and a web browser, can be used to control the simulation. This therefore means that almost every mobile phone (smartphone) and every tablet computer can be used as the external device 72. Thus, this form of communication interface is compatible with a wide range of devices, and also avoids any need to install software.

In the example of Fig. 5, the wireless access point acts as a web server and hosts a network intranet web page or web portal by means of which an external device can establish a communication channel. Thus, in the example of Fig. 5, the peripheral simulation control unit 62 functions as a Wi-Fi web server and hosts a network or intranet page to which the external device 72 can connect using a web-browser. This may for example be an authentication network page, e.g. a log-in intranet page via which authentication credentials can be entered and the secure connection established. Thus, a secure connection interface is facilitated. Furthermore, since connection can only be performed locally, in wireless connection range of the simulation control unit 62, the connection is even more secure.

However, this is not essential and in other examples connection could be established via an internet connection.

Although in the example of Fig. 5, the control input is received from an external device 72 in the form of a smartphone, in further examples, the external device could be a remote device, for instance a remote third party server which is operable to communicate with the simulation control unit to configure parameters of the simulation. For example a communication protocol may be implemented wherein a third party (e.g. a simulation mannequin manufacturer) can control parameters of the simulation. This facilitates for example simultaneous use of the peripheral simulator unit 62 and a simulation mannequin.

An example procedure for establishing a secure communication channel will now be outlined with reference to Fig. 5.

The procedure has two main stages. A first stage is establishing wireless network connection between the external device 72 and the WLAN access point hosted by the peripheral simulation unit 62. The second stage is open a secure communication channel via that wireless network connection, by means of accessing a web server hosted by the peripheral unit and entering authentication credentials.

In the first stage, a series of handshake messages are exchanged for establishing connection. First, the simulation control unit 62 sends an SSID to the external device. The external device then connects to the WLAN access point comprised by the peripheral simulation unit 62 using the SSID. The WLAN access point then sends an acknowledgement message to the external device to confirm the connection. Wireless network connection is then established.

In the second stage, the external device 72 connects to a local web page or intranet page hosted by the web server comprised by the peripheral simulation unit. This comprises the external device seeking access to the login web page ("start page"). Access to the login page is provided by the web server. Access is achieved using a web browser installed on the external device. The user then enters authentication credentials using the web page opened in the browser. These are communicated to the web server. The web server checks the credentials for example using a reference database. If the credentials are authenticated, the web server grants the external device access to a control interface, which is opened in the web browser of the external device. The operator of the external device can then use the control interface on the external device to configure settings and parameters of the simulation mode as discussed above. Control input from the external device is communicated to the web sever of the peripheral simulation unit 62 which then configures the settings and parameters of the simulation accordingly.

Thus, before a remote user can connect to the simulator and simulate any data, an authentication by username and password is needed. A multilayered safety and security approach is thus provided, wherein a password protection is applied to the external login to the web-server, and also wherein a physical connection of the peripheral hardware module 62 is needed to implement the simulation. Thus, a local action and a remote authentication is needed for the simulation to be activated.

Although in the example of Fig. 4 and Fig. 5, the simulation method is implemented by peripheral hardware unit 62 which physically connects with the medical device 12, this not essential and in other examples a fully software based implementation is also possible. For example the medical device may comprise a controller which is configured to implement the simulation mode when activated by a suitable control command, for example from user interface integrated in the medical device. In this case, establishing communication with the external device 72 may be implemented for example by a more direct data communication link (e.g. LAN, WLAN, or USB),

Use of a dedicated peripheral hardware unit 62 may however be preferred since it offers a higher level of security, due to the fact that activation of the simulation mode requires physical connection of the hardware unit to the local medical device, in addition to entry of authentication details remotely using the external device 72. Therefore, an extra layer of security is effectively added.

Furthermore, by using an external peripheral hardware unit 62, the simulation method can be executed without any change to the internal software of the medical device itself. This allows, for instance, updates to the simulation methods to be applied without needing to change or adjust the local software of the medical device itself.

Examples in accordance with a further aspect of the invention provide a system, comprising:
a medical device configured in a standard mode to receive a set of one or more sensor inputs at a communication interface, each carrying a signal representative of a clinical parameter, couple the sensor inputs to a processor, and the processor to generate a user output at a user interface, based on the one or more sensor inputs; and
a controller in accordance with any example or embodiment outlined above, or in accordance with any claim of this application.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method for implementing a simulation mode on a medical device (12),
the medical device being configured in a standard mode to receive a set of one or more sensor inputs (20) at a communication interface (24), each carrying a signal representative of a clinical parameter, to couple the sensor inputs to a processor (14), and the processor configured to generate a user output at a user interface (18), based on the one or more sensor inputs,
the method comprising:
detecting the set of sensor inputs connected at the communication interface;
interrupting the coupling of the sensor inputs to the processor, and
coupling to the processor, in place of the sensor inputs, a set of one or more simulation signals (26), each for simulating a sensor input carrying a particular clinical parameter, wherein the clinical parameters of the simulation signals is selected based at least in part on the detected sensor inputs connected at the communication interface.

2. A method as claimed in claim 1, wherein the clinical parameters simulated by the simulation signals include at least each of the clinical parameters of the detected sensor inputs connected at the connection interface.

3. A method as claimed in claim 1 or 2, wherein the method further comprises interrupting a communication channel between the monitoring device and one or more components of a medical facility monitoring network.

4. A method as claimed in any of claims 1-3, wherein supplying the simulation signals comprises communicating with a datastore, the datastore storing reference simulation data for a plurality of different simulation signals.

5. A method as claimed in claim 4, wherein the simulation data includes historical sensor signal data recorded for one or more prior patients over a time window.

6. A method as claimed in any of claim 4 or 5, wherein the simulation data includes a plurality of simulation data subsets, each simulation data subset comprising simulation signal data for simulating a set of clinical parameters for a particular clinical scenario, over a time window for that clinical scenario.

7. A method as claimed in any of claims 1-6, wherein the medical device is a patient monitor device, for example a fetal monitor device.

8. A method as claimed in any of claims 1-7, wherein the method comprises receiving a control input from an external device and configuring the signal interruption and simulation signal supply based on the control input.

9. A method as claimed in claim 8, wherein the method further includes selectively implementing a further simulation mode responsive to detection of a pre-determined control signal from the external device, wherein, in the further simulation mode, the set of clinical parameters of the simulation signals is selected based on one or more control inputs received from the external device.

10. A method as claimed in claim 8 or 9, wherein the method comprises a step of establishing a communication channel with the external device via a further communication interface, and preferably wherein the establishing communication comprises exchanging one or more handshake messages.

11. A method as claimed in claim 10, wherein the one or more handshake messages include authentication information for establishing a secure communication channel.

12. A method as claimed in claim 10 or 11, wherein the further communication interface includes a local wireless network interface, and preferably wherein the communication channel is facilitated by a local wireless network server, for example a Wi-Fi server.

13. A computer program product comprising code means, configured when run on a processor, to cause the processor to perform the method according to any of claims 1-12.

14. A controller, arranged for communicating with a medical device (12), for implementing a simulation mode on the medical device, the medical device configured in a normal operating mode to receive one or more sensor inputs (20) at a communication interface (24), each carrying a signal representative of a clinical parameter, supply the sensor inputs to a processor (14), and to generate a user output using the processor,
the controller configured to:
communicate with the medical device to cause the medical device to interrupt the coupling of the sensor inputs to the processor of the medical device; and
supply to the processor, in place of the set of sensor input signals, a set of one or more simulation signals (26), each for simulating a sensor input carrying a particular clinical parameter, wherein the clinical parameters of the simulation signals is selected based at least in part on the sensor inputs connected at the communication interface.

15. A controller as claimed in claim 14, wherein the controller is integrated in a peripheral hardware module (62), the hardware module comprising a connection interface, and adapted to operatively couple to the medical device via the connection interface.
